Europäisches Patentamt

European Patent Office

Office européen des brevets

(19)

(11) Numéro de publication: **0 109 335**
**B1**

(12)

# FASCICULE DE BREVET EUROPEEN

(45) Date de publication du fascicule du brevet:
**16.10.85**

(51) Int. Cl.⁴: **C 07 C 11/02**, C 07 C 7/177

(21) Numéro de dépôt: **83402156.0**

(22) Date de dépôt: **07.11.83**

(54) Procédé de prétraitement de coupes d'oléfines légères.

(30) Priorité: **12.11.82 FR 8219009**

(43) Date de publication de la demande:
**23.05.84 Bulletin 84/21**

(45) Mention de la délivrance du brevet:
**16.10.85 Bulletin 85/42**

(84) Etats contractants désignés:
**BE DE GB IT NL**

(56) Documents cités:
**FR - A - 1 346 135**
**US - A - 2 408 010**
**US - A - 2 438 444**
**US - A - 2 777 890**
**US - A - 3 257 473**
**US - A - 3 340 319**

(73) Titulaire: **COMPAGNIE FRANCAISE DE RAFFINAGE**
**Société anonyme dite:, 5, rue Michel-Ange,**
**F-75781 Paris Cedex 16 (FR)**

(72) Inventeur: **Marty, Claude, 66 rue Guillemard, F-76600 Le**
**Havre (FR)**
Inventeur: **Engelhard, Philippe, 197, rue Félix Faure,**
**F-76600 Le Havre (FR)**

(74) Mandataire: **Jolly, Jean-Pierre et al, Cabinet BROT et**
**JOLLY 83, rue d'Amsterdam, F-75008 Paris (FR)**

ACTORUM AG

# Description

La présente invention concerne le prétraitement de charges pétrolières. Elle a plus précisément pour objet un procédé de prétraitement de coupes d'oléfines légères, ou de mélanges de telles coupes, en vue d'en éliminer sélectivement l'isobutène et le butadiène et d'obtenir ainsi une meilleure valorisation de ces coupes d'oléfines.

On sait que le développement contemporain des réactions de craquage des charges pétrolières rend disponibles de grandes quantités d'oléfines possédant de deux à huit atomes de carbone, telles que l'éthylène, le propylène, les divers butènes, pentènes, hexènes, heptènes et octènes. Parmi celles-ci, les coupes d'oléfines à 3 et 4 atomes de carbone, dites coupes $C_3$-$C_4$ et coupes $C_4$, sont particulièrement recherchées, car plusieurs procédés de conversion permettent de les valoriser. En particulier:

— leur oligomérisation permet d'obtenir, après fractionnement, des produits de base intéressants pour la chimie, des solvants ou des huiles lubrifiantes;

— leur alkylation conduit à l'obtention de produits possédant un indice d'octane élevé, qui entrent dans la composition des supercarburants;

— leur dimérisation conduit également à l'obtention de produits possédant un indice d'octane élevé en ce qui concerne les coupes de propylène, et à des oléfines ayant de 6 à 8 atomes de carbone, utiles comme plastifiants, en ce qui concerne les coupes $C_3$-$C_4$, et $C_4$.

Une bonne utilisation de ces coupes d'oléfines nécessite néanmoins une purification préalable, car elles sont sensibles à la présence de nombreux polluants des catalyseurs, tels que le soufre, l'azote, l'isobutène ou le butadiène. Or, s'il est possible d'éliminer au moins partiellement les dérivés soufrés (mercaptans légers, $H_2S$, COS) et azotés (amines légères), il est beaucoup plus difficile d'éliminer l'isobutène et le butadiène, qui produisent des gommes et sont par là responsables de la désactivation rapide des catalyseurs. En outre, la conversion de l'isobutène, par exemple dans les réactions d'oligomérisation, conduit à la formation de composés ramifiés, qui sont généralement hors spécification (heptènes et octènes).

Les divers prétraitements de charges d'oléfines légères proposés jusqu'à présent dans la littérature (par exemple dans la demande de brevet français N° 2 421 157) sont en général très coûteux et peu efficaces. Ils n'éliminent pas complètement l'isobutène et ne permettent pas d'éliminer les traces de butadiène.

En outre, le procédé de prétraitement selon la présente invention permet de traiter les charges d'oléfines à une température beaucoup plus basse, et en particulier à la température ambiante, ce qui permet d'éviter l'isomérisation parasite des butènes normaux en isobutène et d'éviter ainsi de concurrencer la réaction d'élimination des isobutènes objet de la présente invention, au détriment de la charge à traiter.

La présente invention vise donc à proposer un procédé nouveau, simple et économique, qui permette d'éliminer simultanément et de façon sélective, dans des conditions opératoires très peu sévères, l'isobutène et le butadiène des charges pétrolières légères les contenant.

A cet effet, l'invention a pour objet un procédé pour le prétraitement de coupes légères d'oléfines à 3 et 4 atomes de carbone, dites coupes $C_4$ et $C_3$-$C_4$, ou d'un mélange de telles coupes, en vue de l'élimination sélective de l'isobutène et du butadiène présents, ce procédé étant caractérisé en ce que l'on met ladite coupe d'oléfines en contact avec du trifluorure de bore à l'état gazeux en présence d'une masse de remplissage, avec une concentration en trifluorure de bore comprise entre 500 et 10 000 ppm par rapport à la charge à traiter et une vitesse spatiale horaire de la charge comprise entre 0,1 et 10 volumes par volume de masse de remplissage et par heure, et en ce que l'on sépare ensuite de l'effluent, de façon connue en soi, le trifluorure de bore et les polymères d'isobutène et de butadiène obtenus.

Les catalyseurs au trifluorure de bore supportés par de l'alumine sont connus dans la littérature comme catalyseurs de polymérisation des oléfines (brevet français N° 1 346 135). Ils permettent d'effectuer la réaction de polymérisation dans des conditions particulièrement douces, par rapport aux autres catalyseurs de polymérisation, tels que les catalyseurs à l'acide phosphorique ou fluorhydrique.

Dans les réactions d'oligomérisation des coupes pétrolières légères, l'emploi de ces catalyseurs au trifluorure de bore sur alumine est cependant resté limité, du fait de la présence dans les charges à traiter de l'isobutène ou du butadiène, qui donnent des produits indésirables ou qui conduisent à des produits réactionnels hors spécification.

Dans le procédé conforme à l'invention, selon lequel le trifluorure de bore est utilisé à l'état gazeux, on constate cependant que, de façon surprenante, le trifluorure de bore agit sélectivement sur l'isobutène et le butadiène, pour les polymériser, par une réaction de condensation, sans affecter les autres oléfines des coupes $C_4$ et/ou $C_3$-$C_4$.

Le contact entre la charge d'oléfines et le trifluorure de bore peut être effectué dans une enceinte, en présence d'une masse de remplissage inerte, destinée à accroître les surfaces de contact (morceaux de quartz, billes de verre, billes d'alumine, cailloux, etc...) et à éliminer éventuellement les calories produites au cours de la réaction de polymérisation.

Le trifluorure de bore peut être facilement séparé de l'effluent après traitement, par exemple à l'aide d'une distillation flash. Il est préférable de le séparer en premier lieu, afin d'éviter des réactions secondaires avec les oléfines.

Il est ensuite facile de séparer de façon connue, par fractionnement, les polymères d'isobutène et de butadiène qui se sont formés, pour purifier ainsi les coupes $C_3$-$C_4$, ou les coupes $C_4$. Ces charges peuvent être alors soumises à des traitements d'oligomérisation, d'alkylation, de dimérisation ou autres, sans rencontrer les difficultés usuelles de mise en œuvre rappelées ci-dessus et avec une durée d'utilisation beaucoup plus longue des catalyseurs employés.

Les conditions de mise en œuvre du procédé conforme à l'invention peuvent être par exemple les suivantes:

— température: 0 à 100°C,
— pression: 0 à 50 bar.

On notera.que ces conditions opératoires sont peu sévères et, en particulier, que la température peut être égale à la température ambiante ou proche de celle-ci.

Il est ainsi possible d'éliminer sélectivement jusqu'à 95% de l'isobutène et jusqu'à 100% du butadiène présents dans les coupes traitées. Les polymères d'isobutène et de butadiène ainsi séparés des coupes $C_3$-$C_4$ ou $C_4$ sont alors aisément valorisés; en effet, après fractionnement et éventuellement hydrogénation, on obtient, en fonction de leur température d'ébullition:

— des dimères ayant un indice d'octane élevé, utilisables dans les essences légères;
— des trimères intéressants pour la formulation d'essences lourdes, de solvants, ou pour la pétrochimie;
— des tétramères, qui, après hydrogénation, sont des donneurs de bases pour fluides hydrauliques;
— et enfin des polymères plus lourds, qui, également après hydrogénation, sont utilisables comme huile isoparaffinique.

Une telle polymérisation sélective de l'isobutène par rapport aux n-butènes, à partir de mélanges d'oléfines contenues dans les coupes $C_3$-$C_4$ ou $C_4$, n'est pas possible avec les catalyseurs d'oligomérisation usuellement utilisés dans l'industrie, tels que les catalyseurs à base d'esters phosphoriques. En effet, ces catalyseurs étant instables au-dessous de 130°C, un prétraitement des mêmes coupes d'oléfines avec des catalyseurs à base d'esters phosphoriques et sous des conditions opératoires aussi proches que possible de celles utilisées conformément à la présente demande (température du réacteur: 130°C, pression: 40 bar, vitesse spatiale horaire de la charge: 3 vvh) conduirait à des taux de transformation supérieurs à 95% pour l'isobutène, et à 80% pour les n-butènes.

Ainsi qu'il a été indiqué ci-dessus, le prétraitement conforme à l'invention peut être appliqué à toute coupe légère d'oléfines destinée à être soumise à un traitement ultérieur.

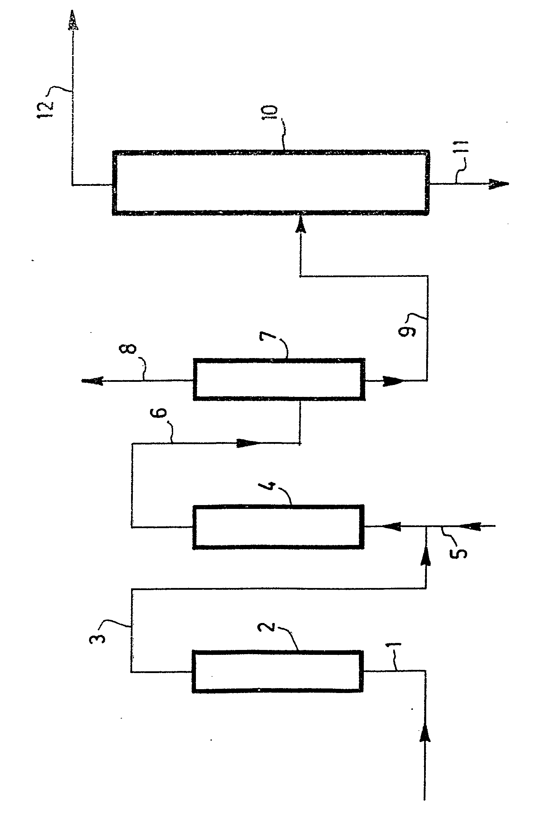

La figure unique du dessin annexé, qui n'a pas de caractère limitatif, est un schéma du procédé de prétraitement de coupes d'oléfines légères conforme à l'invention.

La charge fraîche d'oléfines (coupes $C_3$-$C_4$ et/ou $C_4$) à traiter est introduite par la ligne 1 dans une enceinte 2, où elle est séchée sur des tamis moléculaires, en vue d'éliminer les traces d'humidité susceptibles de réagir avec le trifluorure de bore et de former des borates.

La charge ainsi séchée est conduite par la ligne 3 dans un réacteur 4, après avoir été mélangée avec du trifluorure de bore gazeux, qui emprunte la ligne 5. Compte tenu de la chaleur dégagée par la réaction de l'isobutène ou du butadiène avec le trifluorure de bore, celui-ci pourra avantageusement être dilué dans un gaz inerte tel que l'azote ou l'argon, ou encore dans le butane ou du propane.

En vue d'accroître les surfaces de contact et de retenir la chaleur dégagée par la réaction, le réacteur 4 peut contenir une masse de contact inerte, constituée, par exemple, de morceaux de quartz, de billes de verre, de sable, de billes d'alumine, de cailloux ou autres. Dans les conditions opératoires mentionnées ci-dessus, cette masse de contact peut être maintenue, par exemple, à une température d'environ 20°C.

Les effluents du réacteur 4 sont conduits par la ligne 6 dans un ballon de flash 7, dans lequel le trifluorure de bore gazeux est séparé en tête, par la ligne 8, de la coupe traitée, pour être éventuellement recyclé au réacteur 4. Au fond du ballon de flash 7, la coupe d'oléfines traitée, contenant des polymères de butadiène et d'isobutène, est récupérée par la ligne 9 et acheminée vers une colonne de séparation 10.

On soutire en fond de colonne 10, par la ligne 11, les polymères d'isobutène et de butadiène et, en tête de colonne, par la ligne 12, la coupe $C_3$-$C_4$ ou $C_4$ purifiée, c'est-à-dire débarrassée de l'isobutène et du butadiène indésirables. Les coupes ainsi traitées peuvent être acheminées par la ligne 12 vers des traitements ultérieurs de dimérisation, d'alkylation, d'oligomérisation ou autre, éventuellement après un traitement à la soude destiné à éliminer les traces de trifluorure de bore résiduaire.

Le procédé conforme à l'invention s'applique à toute coupe pétrolière légère en $C_3$-$C_4$ et/ou $C_4$, par exemple à une coupe oléfinique de craquage catalytique.

Les exemples suivants illustrent la mise en œuvre de l'invention.

*Exemple 1*

On traite conformément à l'invention une charge oléfinique venant de craquage catalytique et dont la composition est donnée dans la première colonne du tableau I ci-après.

Les conditions de traitement dans le réacteur 4 sont les suivantes:

| — Température: | 20°C |
| — Pression: | 30 bar |
| — Vitesse spatiale horaire de la charge: | 3 vvh |
| — Débit de trifluorure de bore (rapporté à la charge): | 2000 ppm |
| — Masse de contact: | 100 ml de billes de verre |

L'effluent gazeux sortant de la colonne de séparation 10 (par la ligne 12) a la composition donnée à la seconde colonne du tableau I ci-après:

*(Tableau en tête de la colonne suivante)*

Le prétraitement selon la présente invention a permis un taux d'élimination spécifique de chaque oléfine qui figure au tableau II ci-après:

*Tableau I*

|  | Charge entrée (g) | Effluent sorti (g) |
|---|---|---|
| Hydrocarbures saturés C$_3$/C$_4$ | 56,3 | 56,4 |
| Oléfines | 43,7 | 32,2 |
| dont |  |  |
| Butène 1 | 6,3 | 6,2 |
| Isobutène | 8,8 | 0,5 |
| Butènes 2 | 15,9 | 13,2 |
| Propène | 12,6 | 12,3 |
| Butadiène | 0,06 | 0,02 |
| Polymères formés | 0 | 11,4 |
| Total | 100 | 100 |

*Tableau II*

| Taux d'élimination spécifiques des oléfines (% en poids) | |
|---|---|
| Butène 1 | 1 |
| Isobutène | 94 |
| Butènes 2 | 17 |
| Propène | 2 |
| Butadiène | 70 |

La composition des polymères formés, qui sont extraits par la ligne 11, est la suivante (% en poids) :

```
— Coupe 100-120° C (diisobutène
  et homologues)                    10⎤
— Coupe 120-220° C (triisobutène
  et homologues)                    27 |67%
— Coupe 220-320° C (tétra-isobu-
  tène et homologues)               30⎦
— Polymères lourds 320° C +         33
                                   ────
                                    100
```

## Exemple 2

On traite conformément à l'invention une charge ayant la composition qui figure dans la première colonne du tableau III qui suit.

Cette charge est soumise dans le réacteur 4 à deux séries d'essais, dans les conditions suivantes:
— Température: 20° C,
— Pression: respectivement 2 bar (Essai A) et 4 bar (Essai B),
— Vitesse spatiale horaire de la charge: 3 vvh,
— Débit du trifluorure de bore (rapporté à la charge): 2000 ppm,
— Masse de contact: 100 ml de billes de verre de 2 mm de diamètre.

La composition de l'effluent (Essais A et B) qui sort par la ligne 12 figure dans la seconde et la troisième colonne du tableau III ci-après:

*(Tableau en tête de la colonne suivante)*

Le prétraitement selon l'invention a donné, pour chacun des essai, un taux d'élimination spécifique de chaque oléfine qui apparaît au tableau IV ci-après:

*Tableau III*

|  | Charge entrée (g) | Effluent sorti (g) | |
|---|---|---|---|
|  |  | Essai A | Essai B |
| Hydrocarbures saturés C$_3$/C$_4$ | 56,2 | 56,2 | 56,2 |
| Oléfines | 43,8 | 32,7 | 33,0 |
| dont |  |  |  |
| Butène 1 | 6,4 | 6,3 | 6,4 |
| Isobutène | 8,9 | 0,7 | 0,7 |
| Butènes 2 | 15,8 | 13,3 | 13,4 |
| Propène | 12,7 | 12,4 | 12,5 |
| Butadiène | 0,05 | 0 | 0 |
| Polymères formés | 0 | 11,1 | 10,8 |
| Total | 100 | 100 | 100 |

*Tableau IV*

| Taux d'élimination spécifique de chaque oléfine (% en poids) | | |
|---|---|---|
|  | Essai A | Essai B |
| Butène 1 | 1,5 | 0,5 |
| Isobutène | 92 | 92 |
| Butènes 2 | 16 | 15 |
| Propène | 2,5 | 2 |
| Butadiène | 100 | 100 |

La composition des polymères formés qui sont extraits par la ligne 11 est la suivante (% en poids) :

|  | Essai A | | Essai B | |
|---|---|---|---|---|
| — Coupe 100-120° C (diisobutène et homologues) | 13⎤ |  | 12⎤ |  |
| — Coupe 120-220° C (triisobutène et homologues) | 26 | 65 | 28 | 66 |
| — Coupe 220-320° C (tétra-isobutène et homologues) | 26⎦ |  | 26⎦ |  |
| — Polymères lourds 320° C + | 35 |  | 34 |  |

L'invention apporte donc un procédé simple et facile à mettre en œuvre, du fait de ses conditions opératoires peu sévères, pour éliminer la plus grande partie de l'isobutène et du butadiène contenus dans une coupe pétrolière légère préalablement à un traitement de valorisation (oligomérisation, dimérisation, alkylation ou autre) des oléfines contenues dans cette coupe.

## Revendications

1. Procédé pour le prétraitement de coupes légères d'oléfines à 3 et 4 atomes de carbone, dites coupes C$_4$ et C$_3$-C$_4$, ou d'un mélange de telles coupes, en vue de l'élimination sélective de l'isobutène et du butadiène présents, ce procédé étant caractérisé en ce que l'on met ladite coupe d'oléfines en contact avec du trifluorure de bore à l'état gazeux, en présence d'une masse de remplissage, avec une concentration en trifluorure de bore comprise entre 500 et 10 000 ppm par rapport à la charge à traiter et une vitesse spatiale horaire de la charge comprise entre 0,1 et 10 volumes par volume de masse de remplissage et par heure, et en ce que l'on sépare ensuite de l'effluent, de façon connue en soi, le trifluorure de bore et les polymères d'isobutène et de butadiène obtenus.

2. Procédé selon la revendication 1, caractérisé en ce que ladite coupe d'oléfines et le trifluorure de bore gazeux sont mis en contact à une température comprise entre 0 et 100° C et sous une pression comprise entre 0 et 50 bar.

3. Procédé selon l'une des revendications 1 ou 2, caractérisé en ce que le trifluorure de bore est séparé de la coupe prétraitée, préalablement à la séparation des polymères d'isobutène et du butadiène.

4. Procédé selon la revendication 3, caractérisé en ce que la séparation du trifluorure de bore de l'effluent comprend une phase de distillation flash.

5. Procédé selon l'une des revendications 1 à 4, caractérisé en ce que la séparation des polymères de butadiène et d'isobutène de l'effluent s'effectue par fractionnement.

6. Procédé selon l'une des revendications 1 à 5, caractérisé en ce que, après séparation du trifluorure de bore et des polymères formés, l'effluent est soumis à un traitement à la soude en vue d'éliminer les traces résiduaires du trifluorure de bore.

7. Procédé selon l'une des revendications 1 à 6, caractérisé en ce que le trifluorure de bore séparé de l'effluent est recyclé au prétraitement de la coupe d'oléfines.

## Patentansprüche

1. Verfahren zur Vorbehandlung von leichten Fraktionen von Olefinen mit 3 und 4 Kohlenstoffatomen, sogenannten $C_4$- und $C_3$-$C_4$-Fraktionen, oder eines Gemisches solcher Fraktionen zum Zwecke der selektiven Eliminierung von vorhandenem Isobuten und Butadien, dadurch gekennzeichnet, dass die Fraktion von Olefinen mit Bortrifluorid in gasförmigem Zustand in Gegenwart einer Füllmasse mit einer Bortrifluoridkonzentration zwischen 500 und 10 000 ppm, bezogen auf die zu behandelnde Charge, und mit einer Chargenvolumengeschwindigkeit zwischen 0,1 und 10 Volumen pro Volumen Füllmasse und pro Stunde in Berührung gebracht wird, und dass anschliessend das Bortrifluorid und die erhaltenen Isobuten- sowie Butadienpolymerisate vom Ausfluss auf an sich bekannte Art und Weise abgetrennt werden.

2. Verfahren nach Anspruch 1, dadurch gekennzeichnet, dass die Fraktion von Olefinen und das gasförmige Bortrifluorid bei einer Temperatur zwischen 0° C und 100° C und unter einem Druck zwischen 0 bar und 50 bar miteinander in Berührung gebracht werden.

3. Verfahren nach Anspruch 1 oder 2, dadurch gekennzeichnet, dass das Bortrifluorid vor der Abtrennung der Isobuten- und Butadienpolymerisate von der vorbehandelten Fraktion abgetrennt wird.

4. Verfahren nach Anspruch 3, dadurch gekennzeichnet, dass die Abtrennung des Bortrifluorids vom Ausfluss eine Flashdestillationsphase umfasst.

5. Verfahren nach einem der Ansprüche 1 bis 4, dadurch gekennzeichnet, dass die Abtrennung der Butadien- und Isobutenpolymerisate vom Ausfluss durch Fraktionieren erfolgt.

6. Verfahren nach einem der Ansprüche 1 bis 5, dadurch gekennzeichnet, dass der Ausfluss nach der Abtrennung des Bortrifluorids und der gebildeten Polymerisate einer Sodabehandlung unterworfen wird, um die restlichen Bortrifluoridspuren zu eliminieren.

7. Verfahren nach einem der Ansprüche 1 bis 6, dadurch gekennzeichnet, dass das vom Ausfluss abgetrennte Bortrifluorid zur Vorbehandlung der Fraktion von Olefinen rückgeführt wird.

## Claims

1. A process for the pretreatment of light olefin fractions having 3 and 4 carbon atoms, known as $C_4$ and $C_3$-$C_4$ fractions, or a mixture of these fractions, with a view to the selective elimination of the isobutene and butadiene present, this process being characterized in that said olefin fraction is brought into contact with boron trifluoride in the gaseous state, in the presence of a filling material, with a concentration of boron trifluoride of between 500 and 10 000 ppm in relation to the charge to be treated and at an hourly spatial velocity of the charge of between 0.1 and 10 volumes per volume of filling material and per hour, and in that subsequently the boron trifluoride and the polymers of isobutene and butadiene obtained are separated from the effluent in *per se* known manner.

2. A process according to Claim 1, characterized in that said fraction of olefins and the gaseous boron trifluoride are brought into contact at a temperature of between 0 and 100° C and at a pressure of between 0 and 50 bar.

3. A process according to either of Claims 1 and 2, characterized in that the boron trifluoride is separated from the pretreated fraction, prior to the separation of the isobutene and butadiene polymers.

4. A process according to Claim 3, characterized in that the separation of boron trifluoride from the effluent comprises a flash distillation stage.

5. A process according to any of Claims 1 to 4, characterized in that the separation of the butadiene and isobutene polymers from the effluent is carried out by fractionation.

6. A process according to any of Claims 1 to 5, characterized in that, after separation of boron trifluoride and the polymers formed, the effluent is subjected to a treatment with soda so as to eliminate residual traces of boron trifluoride.

7. A process according to any of Claims 1 to 6, characterized in that the boron trifluoride separated from the effluent is recycled to the pretreatment of the fraction of olefins.

0 109 335